# EUROPÄISCHE PATENTSCHRIFT

(11) **EP 2 162 061 B1**
(45) Veröffentlichungstag und Bekanntmachung des Hinweises auf die Patenterteilung: **02.05.2012**
(21) Anmeldenummer: 08735360.3
(22) Anmeldetag: 22.04.2008
(51) Int. Cl.: A61B 5/00

(54) **NACHWEIS EINER APNOE MIT BLUTDRUCKABHÄNGIG ERFASSTEN SIGNALEN**
DETECTION OF AN APNOEA BY MEANS OF A SIGNAL DEPENDENT ON BLOOD PRESSURE
DÉTECTION D'UNE APNÉE À L'AIDE DE SIGNAUX ACQUIS EN FONCTION DE LA PRESSION ARTÉRIELLE

(30) Priorität: 27.04.2007 DE 102007020038
(43) Veröffentlichungstag der Anmeldung: 17.03.2010
(73) Patentinhaber: Fraunhofer-Gesellschaft zur Förderung der angewandten Forschung e.V., 80686 München (DE)
(72) Erfinder: MOERSDORF, Hans-Joachim, 90763 Fürth (DE); ASCHENBRENNER, Stefan, 90542 Eckental (DE); HOFMANN, Christian, 90489 Nürnberg (DE)
(74) Vertreter: Zimmermann, Tankred Klaus
(86) Internationale Anmeldenummer: PCT/EP2008/003232
(87) Internationale Veröffentlichungsnummer: WO 2008/135161

(56) Entgegenhaltungen:
- EP-A- 1 470 780
- WO-A-98/04182
- PENZEL T ET AL: "Peripheral arterial tonometry monitors changes of autonomous nervous system in sleep apnea" SECOND JOINT EMBS-BMES CONFERENCE 2002. CONFERENCE PROCEEDINGS. 24TH. ANNUAL INTERNATIONAL CONFERENCE OF THE ENGINEERING IN MEDICINE AND BIOLOGY SOCIETY. ANNUAL FALL MEETING OF THE BIOMEDICAL ENGINEERING SOCIETY. HOUSTON, TX, OCT. 23 - 26, 2002; [ANN, Bd. 2, 23. Oktober 2002 (2002-10-23), Seiten 1552-1553, XP010620207 ISBN: 978-0-7803-7612-0

## Beschreibung

Die vorliegende Erfindung befasst sich mit dem Nachweis von Schlafstörungen und insbesondere damit, wie mittels blutdruckabhängig erfasster Signale eine Apnoe nachgewiesen werden kann.

Schlafstörungen sind ein immer häufiger auftretendes Phänomen, das die Lebensqualität und die Leistungsfähigkeit der betroffenen Personen stark einschränkt. Bei speziellen Arten von auftretenden Schlafstörungen kann darüber hinaus die Gesundheit des Patienten nachhaltig beeinträchtigt werden.

Zwei besonders häufig auftretende Schlafstörungen sind dabei Apnoen und Hypopnoen. Bei der Apnoe treten vollständige Atemstillstände auf, deren Häufigkeit in weiten Grenzen variieren kann, wobei Werte von über 200-300 solcher Schlafstörungen pro Nacht keine Seltenheit sind. Als allgemeine Definition für das Krankheitsbild der Apnoe gilt das Auftreten von mindestens 10 Atemstillständen, welche jeweils mindestens 10 Sekunden dauern, innerhalb einer Schlafstunde. Die Apnoe kann mehrere Ursachen haben, die häufigste ist ein während des Schlafes auftretender Verschluss der oberen Atemwege (obstruktive Schlaf-Apnoe). Der Verschluss wird normalerweise durch einen Kollaps der Luftröhre oder durch ein Verschließen der Luftröhre mit der Zunge ausgelöst. Auch das Velum kann beteiligt sein, welches unter anderem auch für das Schnarchen verantwortlich ist. Erschlafft das Gaumensegel, kann es dazu führen, dass dieses die Atemwege vollständig verschließt, so dass die Sauerstoffzufuhr zu den Lungen und somit auch zum Gehirn unterbrochen wird. Aufgrund obigen Zusammenhangs wird die Apnoe auch häufig bei Personen beobachtet, die zu starkem Schnarchen neigen.

Der abfallende Sauerstoffgehalt des Blutes löst nach einer gewissen Zeit ein Alarmsignal bzw. eine Gegenmaßnahme im Gehirn aus, so dass die betroffenen Personen am Ende einer Apnoe eine kurzzeitige zentralnervöse Aktivierung, ein sog. Arousal, erleben (vergleichbar einer Panikreaktion). Beim Arousal schreckt der betroffene Patient typischerweise mit einem lauten Schnarchgeräusch hoch, woraufhin die Atmung wieder einsetzt. Der Sauerstoffgehalt kann sich wieder normalisieren. Da sich, wie oben beschrieben, dieser Vorgang mehrmals pro Nacht wiederholt, wird offensichtlich, dass die Schlaf-Apnoe eine Reihe von negativen Begleiterscheinungen hervorrufen kann, wie beispielsweise verstärkte Tagesmüdigkeit, verminderte geistige und körperliche Leistungsfähigkeit, Konzentrationsschwäche, Kopfschmerzen, Depressionen und dergleichen.

Neben der obstruktiven Apnoe wird häufig auch die sog. zentrale Schlaf-Apnoe beobachtet, bei der kein Verschluss der Atemwege erfolgt, sondern die vielmehr auf ein Aussetzen der Atemimpulse seitens des Gehirns zurückzuführen ist. Dabei ist der beobachtbare Ablauf der Apnoe bis hin zum Arousal im Wesentlichen derselbe wie bei der obstruktiven Apnoe.

Zusammengefasst lässt sich das Krankheitsbild der Schlafapnoe durch wiederkehrende Atemstillstände während des Schlafs kennzeichnen. Dabei lassen sich Schlafapnoen bzw. Apnoen, wie bereits erwähnt, grob in zwei Gruppen untergliedern: die zentralen Apnoen und die obstruktiven Apnoen. Bei der zentralen Apnoe kommt es im Gehirn zu Fehlern bei der Aktivierung der Atmungsmuskulatur und bei der obstruktiven Apnoe führen Verlegungen im Rachen- und Halsraum zu einem Verschluss der Atemwege. Die Detektion von Apnoen erfolgt üblicherweise, indem mehrere Vitalparameter eines zu untersuchenden Patienten während des Schlafes aufgezeichnet werden, wie zum Beispiel die Atmungsanstrengung, der Atmungsfluss, oder die Blutsauerstoffsättigung. Auch anhand eines EEG kann eine Apnoe erkannt werden. Da eine Apnoe nur schwierig nachgewiesen werden kann, müssen typischerweise mehrere der oben beschriebenen Vitalparameter in Kombination betrachtet werden, um zu einer eindeutigen Aussage zu gelangen. Einige Verfahren können eine Apnoe auch detektieren, wenn lediglich eine Teilmenge der obigen Vitalparameter beziehungsweise eine geringere Anzahl von Vitalparametern aufgezeichnet wird.

Aufgrund der hohen Anzahl von auszuwertenden Parametern beziehungsweise der Komplexität der Auswertung, können Apnoen normalerweise erst nach deren Ende, also nachdem ein Arousal die Apnoe beendet hat, nachgewiesen werden. Die Ursachen hierfür liegen zum einen in der prinzipiellen Schwierigkeit, den Beginn einer Apnoe in den aufgezeichneten Vitalparametern zu erkennen beziehungsweise in der erforderlichen hohen Signalverarbeitungskomplexität.

Die EP 1 470 780 A1 betrifft eine Vorrichtung und ein Verfahren zur Diagnose einer Schlafapnoe, wobei Licht an ein bestimmtes Körperteil gerichtet wird und die ausgegebenen Lichtsignale zur Bestimmung einer Apnoe ausgewertet werden. Die Gleichstromkomponenten des PPG-Signalverlaufs werden betrachtet, und hier insbesondere die Gleichstromanteile der Ausgangssignale, die für die verschiedenen Wellenlängen (rotes Licht und infrarotes Licht) erhalten werden. Diese Gleichstromkomponenten werden in Relation zueinander gesetzt, genauer gesagt werden die Gleichstromkomponenten des roten Lichtes und des infraroten Lichtes verwendet, wobei ein Teiler vorgesehen ist, der die Gleichstromkomponente des infraroten Lichtes durch die Gleichstromkomponente des roten Lichtes teilt, so dass sich der Verhältnisindex RI ergibt, der dann mit einem durch eine Steuerung bereitgestellten Bezugswert verglichen wird Basierend auf einem Vergleich des Verhältnisindex RI mit dem Bezugswert kann festgestellt werden, ob eine Apnoesituation vorliegt oder nicht. Genauer gesagt wird davon ausgegangen, dass ein Wert von RI größer als ein Differenzwert auf einen Apnoezustand hinweist. Die mittleren Werte für den Index RI werden für sechs Testpersonen während eines normalen Atmungszustands sowie während eines Apnoezustandes erfasst. Ein Wert für den Bezugswert des Komparators wird basierend auf der Testfolge bestimmt.

Der vorliegenden Erfindung liegt die Aufgabe zugrunde, einen verbesserten Ansatz zum Nachweis von Apnoen zu schaffen, der insbesondere auch dann zuverlässige Informationen liefert, wenn das betrachtete Pulswellensignal Änderungen unterworfen ist, die auf Faktoren zurückgehen, die nicht mit der Apnoe zusammenhängen.

Diese Aufgabe wird durch eine Vorrichtung gemäß Anspruch 1 und durch ein Verfahren nach Anspruch 14 gelöst.

Die Auswertung von photoplethysmografisch erfassten Pulswellen erlaubt dabei auf deutlich günstigere, effizientere und schnellere Art und Weise, eine Apnoe nachzuweisen. Wie aus der eingehenderen Diskussion der Ausführungsbeispiele der Erfindung in den folgenden Absätzen hervorgehen wird, ist es dabei sogar möglich, den Beginn einer Apnoe zu detektieren. Einige Ausführungsbeispiele der vorliegenden Erfindung machen sich dies zunutze, um eine Alarmaktion auszuführen, die beispielsweise beinhalten kann, dass ein Patient, bei dem der Beginn einer Apnoe nachgewiesen wurde, stimuliert wird. Durch den rechtzeitigen Stimulus kann erreicht werden, dass die Apnoe vorzeitig beendet wird, bevor der Körper in eine hohe Sauerstoffschuld gelangt.

Gemäß einem bevorzugtem Ausführungsbeispiel der vorliegenden Erfindung wird als Alarmmaßnahme ein CPAP- (Continuous Positive Airway Pressure) Apparat aktiviert bzw. es wird der Druck, mit dem dieser Apparat arbeitet, erhöht. CPAP-Geräte sind zur Therapie von Apnoen verwendete Geräte, bei denen der Patient eine Maske trägt, die mit dem CPAP-Gerät verbunden ist, welches dafür sorgt, dass der Patient kontinuierlich beatmet wird bzw. dass in der dem Patienten zugeführten Atemluft ständig ein fest vorgegebener Luftdruck herrscht. Dies kann einen Kollaps der Luftröhre verhindern. Die herkömmlichen CPAP-Geräte haben eine Reihe von unangenehmen Nebenwirkungen, obwohl sie prinzipiell die Apnoe-Häufigkeit deutlich verringern können.

Zum Einen wird durch den ständigen Betrieb des CPAP-Geräts die Luftröhre ausgetrocknet, zum Anderen erleiden die Patienten häufig eine Bindehautentzündung, da es nicht möglich ist, die zur Beatmung erforderliche Maske vollständig dicht am Patienten anliegen zu lassen. Durch den im CPAP-Gerät erzeugten Überdruck entweicht somit ständig Luft an den Rändern der Atemmaske, unter anderem auch in Richtung der Augen, was zu der oben beschriebenen Bindehautentzündung führen kann. Bei einem Ausführungsbeispiel der Erfindung wird bei nachgewiesener Apnoe das CPAP-Gerät dann aktiviert, wenn es wirklich erforderlich ist. Dabei kann entweder das CPAP-Gerät erst zu diesem Zeitpunkt eingeschaltet werden oder es kann alternativ ein mit geringem Druck (also auch mit geringen Nebenwirkungen) arbeitendes CPAP-Gerät derart gesteuert werden, dass kurzzeitig der Druck erhöht wird. Durch ein System aus CPAP-Gerät und einer Vorrichtung zum Nachweis von Apnoen können also die auftretenden Nebenwirkungen beim Einsatz von CPAP-Geräten deutlich verringert oder vermieden werden.

Bei einem weiteren Ausführungsbeispiel kann der zu analysierende niederfrequente Anteil des photoplethysmografisch erfassten Pulswellensignals durch Tiefpassfilterung mit einer Grenzfrequenz, die unterhalb der durchschnittlichen Pulsfrequenz liegt, erzeugt werden.

Bei weiteren bevorzugtem Ausführungsbeispielen der vorliegenden Erfindung wird ein einfacher Schwellwertvergleich dazu verwendet, eine Apnoe oder vielmehr den Beginn einer Apnoe nachzuweisen, so dass insgesamt nur eine Signalverarbeitung geringer Komplexität erforderlich ist.

Bei einem weiteren bevorzugtem Ausführungsbeispiel der Erfindung kann die Grenzfrequenz des Tiefpassfilters adaptiv an den Puls beziehungsweise die Pulsfrequenz des beobachteten Patienten angepasst werden, so dass zu jeder Zeit sichergestellt ist, dass das auszuwertende Signal nicht durch die typischerweise zeitlich variierende Pulsfrequenz beeinträchtigt werden kann.

Bei einem weiteren bevorzugtem Ausführungsbeispiel der vorliegenden Erfindung kann auf einfache Art und Weise ein Maß für die Schwere oder Heftigkeit einer Apnoe abgeleitet werden, indem diese in Abhängigkeit von der Differenz zum momentan gültigen Schwellwert bestimmt wird.

In ähnlicher Art und Weise kann bei einem weiteren Ausführungsbeispiel eine aufgetretene Apnoe von einer Hypopnoe unterschieden werden. Ein starkes Unterschreiten des momentan gültigen Schwellwerts deutet auf das Vorliegen einer Apnoe hin, während eine leichte Unterschreitung desselben das Vorliegen einer Hypopnoe anzeigen kann. Demzufolge kann ein optional verbundenes CPAP-Gerät beispielsweise bei Vorliegen einer Hypopnoe zunächst ausgeschaltet bleiben, da es bei einer Hypopnoe länger dauert als bei einer Apnoe, bis der Patient eine kritische Sauerstoffschuld eingeht.

Bei einem weiteren Ausführungsbeispiel wird ein vereinfachter photoplethysmografischer Sensor verwendet, um die photoplethysmografischen Pulswellen aufzuzeichnen, welcher lediglich mit Licht eines einzigen Wellenlängenbereichs arbeitet. Typische photoplethysmografische Sensoren verwenden zwei Wellenlängenbereiche parallel, so dass bei diesem Ausführungsbeispiel die Materialkosten beziehungsweise die Kosten der verwendeten Hardware weiter gesenkt werden können.

Bevorzugte Ausführungsbeispiele der vorliegenden Erfindung werden nachfolgend, bezugnehmend auf die beiliegenden Figuren, erläutert. Es zeigt:
- Fig. 1: ein photoplethysmografisch aufgezeichnetes Pulswellensignal;
- Fig. 2: eine schematische Analyse eines niederfrequenten Signalanteils;
- Fig. 3: ein Beispiel einer erfindungsgemäßen Vorrichtung zum Nachweis von Apnoen;
- Fig. 4: ein weiteres Beispiel einer Vorrichtung zum Nachweis von Apnoen; und
- Fig. 5: ein Beispiel eines Verfahrens zum Nachweis von Apnoen.

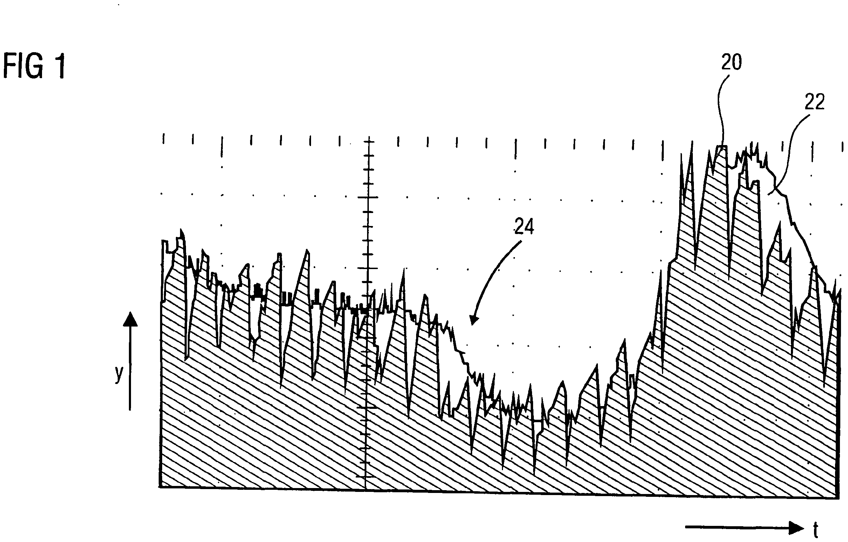

Anhand von Fig. 1 wird im Folgenden die photoplethysmografische Erfassung von Messdaten kurz dargestellt und basierend darauf die einigen Implementierungen der Erfindung zugrunde liegenden Ideen entwickelt.

Fig. 1 zeigt auf der x-Achse die Zeit t in willkürlichen Einheiten und auf der y-Achse eine mittels eines photoplethysmografischen Sensors aufgenommene Lichtintensität in ebenfalls willkürlichen Einheiten.

Photoplethysmografische Erfassung von Pulswellen wird häufig zur nicht-invasiven Messung des Blutsauerstoffgehalts (SpO₂) angewendet. Dazu wird ein Körperteil - häufig ein Finger, manchmal jedoch auch andere Körperteile wie Ohrläppchen, Handgelenk oder Sternum - mittels einer modulierten Lichtquelle durchstrahlt. Bei der Bestimmung der Blutsauerstoffsättigung wird normalerweise Licht mit zwei unterschiedlichen Wellenlängen verwendet.

In anderen Anwendungsgebieten werden auch mehr als zwei unterschiedliche Wellenlänge verwendet, in einigen speziellen einfachen Anwendungsfällen kann eine einzelne Wellenlänge zum Einsatz kommen.

Generell wird bei der Photoplethysmografie also ein Körperteil mit Licht durchstrahlt, wobei die Intensität der nach der Durchstrahlung des Körperteils empfangbaren Lichtmenge als Messgröße bestimmt wird.

Fig. 1 zeigt in der gezackten Kurve 20 den mittels eines Oszillographen dargestellten Verlauf eines photoplethysmografisch erfassten Pulswellensignals, also im Wesentlichen eine erfassten Lichtintensität einer Durchlichtaufnahme eines Körperteils. Dabei ist zu bemerken, dass die Lichtkurve einer zweiten normalerweise verwendeten Lichtquelle in Fig. 1 aus Gründen der Darstellbarkeit nicht gezeigt ist. Die glatte Kurve 22 zeigt einen niederfrequenten Anteil eines Pulswellensignals, wie er beispielsweise durch Tiefpassfilterung des gezackten Signals 20 erzeugt werden kann.

Die gezackte Kurve 20 stellt somit gewissermaßen die Kurve der aktuellen Helligkeit dar, wie sie durch eine Kombination eines Gleichspannungsanteils 22 und eines dynamischen Anteils gebildet werden kann. Die einzelnen Zacken der gezackten Kurve 20 entsprechen den einzelnen Pulsschlägen des Patienten, die im vorliegenden Beispiel an einem Finger des Patienten beobachtet wurden. Dieser spezifische Kurvenverlauf entsteht dadurch, dass mit jedem Pulsschlag kurzfristig der Blutdruck im Adernsystem des Menschen ansteigt, wodurch sich der Durchmesser der Adern geringfügig erhöht. Diese Variation des Durchmessers führt dazu, dass das die Blutgefäße durchstrahlende Licht eine längere optische Weglänge durch das Blut zurücklegen muss, was zur verstärkten Absorption führt, so dass jeder Pulsschlag einen kurzzeitigen Intensitätseinbruch des photoplethysmografisch nachgewiesenen Lichts beziehungsweise der nachgewiesenen Intensität zur Folge hat. Die glatte Kurve 22 entsteht durch zeitliche Mittelung des dynamischen Signals, also der gezackten Kurve 20 und zeigt somit dessen Gleichspannungsanteil (DC-Komponente). Dabei ist es für einige Ausführungsbeispiele der Erfindung ausreichend, eine vereinfachte Version eines SpO₂-Plethysmografen zu verwenden, da lediglich eine einzelne Messkurve für Licht einer einzelnen Wellenlänge ausgewertet werden muss, um zuverlässig auf eine Apnoe zu schließen.

Der Gleichspannungsanteil beziehungsweise der niederfrequente Signalanteil 22 ändert sich bei einer photoplethysmografischen Messung im Laufe der Zeit durch die Änderung einiger die Messung beeinflussenden Parameter, wie beispielsweise sich änderndes Umgebungslicht, eine Lageänderung des Messorts relativ zum Blutgefäß, der Höhe des Messortes über dem Herzen und sich ändernder Schlafpositionen des zu untersuchenden Patienten. Darüber hinaus weist dieser starke interindividuelle Streuung auf, ist also extrem patientenabhängig.

Von den Erfindern wurde erkannt, dass bei einer Obstruktion, wie sie beispielsweise durch Atmen bei verschlossener Nase und Mund herbeiführbar ist, ein spontaner Anstieg des Blutdrucks als Reaktion auf die intrathorakalen Druckschwankungen beobachtet werden kann. Da die zugrundeliegenden Mechanismen der Atemstörungen ähnlich sind, gilt dies auch für zentrale Apnoen.

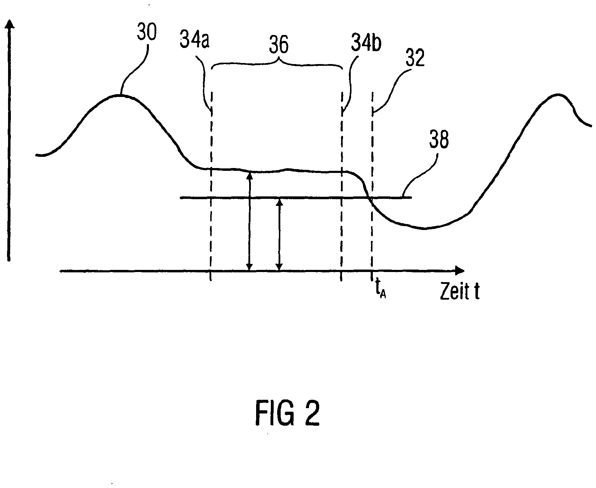

Bei Aussetzen der Atmung in Folge einer Apnoe erfolgt also ein spontaner Anstieg des Blutdrucks, wobei dies auf einer Zeitskala erfolgt, die sich deutlich von der den Pulsschlägen zugrunde liegenden Zeitskala unterscheidet. Treten solche durch Apnoen verursachte Blutdruckänderungen auf, resultieren diese ebenfalls in einer Weitung der Gefäße, womit der von den Lichtstrahlen zu passierende Querschnitt zunimmt. Infolgedessen kommt es zu einer Verschiebung des Gleichspannungsanteils in der Helligkeitskurve, wie es in Fig. 1 zum Zeitpunkt des Beginns einer Apnoe 24 dargestellt ist, an dem der niederfrequente Anteil des Pulswellensignals deutlich einbricht. Der zum Zeitpunkt 24 eintretende Abfall des niederfrequenten Anteils des Pulswellensignals erlaubt also unmittelbar, auf das Vorhandensein einer Apnoe zu schließen. Dabei ist zu berücksichtigen, dass beobachtete Änderungen des Gleichspannungsanteils auch durch die bereits genannten Störeinflüsse hervorgerufen werden können. Jedoch können diese beiden Änderungen durch äußerst einfache Signalverarbeitungsmethoden, wie beispielsweise die anhand von Fig. 2 beschriebenen Schwellwertvergleiche, unterschieden werden, so dass eine Apnoendetektion und insbesondere sogar die Detektion des Beginns einer Apnoe mit äußerst einfachen Mitteln in Echtzeit erfolgen kann.

Wie anhand der oben beschriebenen medizinischen Mechanismen klar wird, wird bei einigen Ausführungsbeispielen der Erfindung ein blutdruckabhängiges Pulswellensignal bereitgestellt. Das Pulswellensignal wird ausgewertet, um auf das Vorhandensein einer Apnoe zu schließen, wenn eine Änderung eines Signalverlaufs des Pulswellensignals ein vorbestimmtes Nachweiskriterium erfüllt.

Bei weiteren Ausführungsbeispielen der vorliegenden Erfindung können Sensoren zum Einsatz kommen, die auf andere Art und Weise ein blutdruckabhängiges Pulswellensignal zur Verfügung stellen. Dies können beispielsweise hoch empfindliche Blutdruckmessgeräte bzw. integrierte Drucksensoren sein. Darüber hinaus kann beispielsweise die Information eines EEG oder eine EKG verwendet werden, da diese ebenfalls Informationen über den Verlauf des Blutdrucks beinhalten. Bei weiteren Ausführungsbeispielen welche nicht erfindungsgemäβ sind, kann auch ein hochfrequenterer Signalanteil des Pulswellensignals ausgewertet werden, um auf das Vorhandensein einer Apnoe zu schließen.

Motiviert kann eine solche Vorgehensweise beispielsweise dadurch sein, dass bei Vorliegen einer Sauerstoffschuld die Elastizität der Adern im menschlichen Körper verringert ist. Demzufolge wird ein Druckanstieg, wie er durch den Herzschlag hervorgerufen wird, weniger stark durch elastische Reaktionen des Adersystems gedämpft. Demzufolge wird sich auch also ein höherfrequenter Signalanteil des blutdruckabhängigen Signals (die Zacken der gezackten Kurve 20 in Fig. 1) ändern. Dies rührt daher, dass der Druckanstieg wesentlich schneller erfolgt, d.h. die Steigung der Zacken, bzw. die Steigung der Signalflanken eines einen Pulsschlag beschreibenden Signalanteils eines blutdruckabhängigen Pulswellensignals wird sich gegenüber einem elastischen Gefäßsystem erhöhen. Demzufolge können alternative Ausführungsbeispiele auf Grundlage dieser Information auch eine Änderung eines höherfrequenten Signalanteils des Pulswellensignals auswerten, um aus einer Änderungsgeschwindigkeit des höherfrequenten Signalanteils auf das Vorhandensein einer Apnoe zu schließen.

Dazu zeigt Fig. 2 schematisch einen Kurvenverlauf eines niederfrequenten Anteils eines Pulswellensignals 30, wobei der Signalverlauf in willkürlichen Einheiten der Intensität gegen die Zeit in willkürlichen Einheiten aufgetragen ist.

Fig. 2 zeigt ferner einen Auswertezeitpunkt 32 (t_{A}), sowie einen Intervallstartzeitpunkt 34a und einen Intervallendzeitpunkt 34b, die ein Zeitintervall 36 definieren, welches zeitlich vor dem Auswertezeitpunkt 32 liegt. Der Nachweis einer Apnoe und insbesondere auch das Beginnen einer Apnoe zum Auswertezeitpunkt 32 kann nunmehr dadurch erfolgen, dass beispielsweise ein absolutes Schwellwertkriterium definiert wird, dass also bei Unterschreiten eines festen Schwellwerts 38 des niederfrequenten Anteils des Pulswellensignals auf das Vorhandensein einer Apnoe beziehungsweise auf den Beginn einer Apnoe geschlossen wird.

Bei der vorliegenden Erfindung, die zusätzlich der Tatsache Rechnung trägt, dass sich der niederfrequente Signalanteil aufgrund anderer Faktoren ebenfalls ändern kann, wird ein zeitlich variabler Schwellwert 38 dadurch gebildet, dass das Signal innerhalb des Zeitintervalls 36, welches sich zeitlich vor dem Auswertezeitpunkt 32 befindet, zur Berechnung des Schwellwerts 38, unterhalb dessen auf das Vorhandensein einer Apnoe geschlossen wird, verwendet wird. Dies kann beispielsweise dadurch geschehen, dass der Mittelwert des Pulswellensignals innerhalb des Intervalls 36 gebildet wird, woraufhin der Schwellwert 38 beispielsweise als fester Bruchteil des innerhalb des Intervalls 36 gebildeten Mittelwerts festgelegt wird. Dabei kann das Zeitintervall 36 zum einen, wie es in Fig. 2 gezeigt ist, zeitlich so vor dem Auswertezeitpunkt 38 liegen, dass das Zeitintervall 36 nicht bis zum Auswertezeitpunkt 32 dauert. Alternativ ist es natürlich ebenfalls möglich, ein Zeitintervall zu wählen, welches mit dem Auswertezeitpunkt 32 endet. Alternative Ausführungsbeispiele können beispielsweise auch die zeitliche Ableitung des niederfrequenten Anteils des Pulswellensignals als Kriterium verwenden, so dass, wenn die zeitliche Ableitung unterhalb einen bestimmten Wert fällt, auf das Vorhandensein einer Apnoe geschlossen werden kann.

Alternativ ist es beispielsweise auch möglich, innerhalb des Zeitintervalls 36 eine lineare Regression durchzuführen oder ein Polynom höherer Ordnung an den Signalverlauf anzupassen, um ein Nachweiskriterium, bei dessen Erfüllung auf das Vorhandensein einer Apnoe geschlossen wird, zum Auswertezeitpunkt 32 auf Grundlage der zum Auswertezeitpunkt 32 extrapolierten angepassten Funktion zu bestimmen. Vorteilhaft ist bei allen oben beschriebenen Auswerte- beziehungsweise Analyseverfahren, dass aufgrund des photoplethysmografisch gewonnenen Pulswellensignals die Auswerteschritte beziehungsweise die zur Signalverarbeitung erforderlichen Verfahrensschritte ohne großen Rechenaufwand durchführbar sind, so dass die Apnoen zum einen mit günstiger Hardware und zum anderen in Echtzeit nachgewiesen werden können.

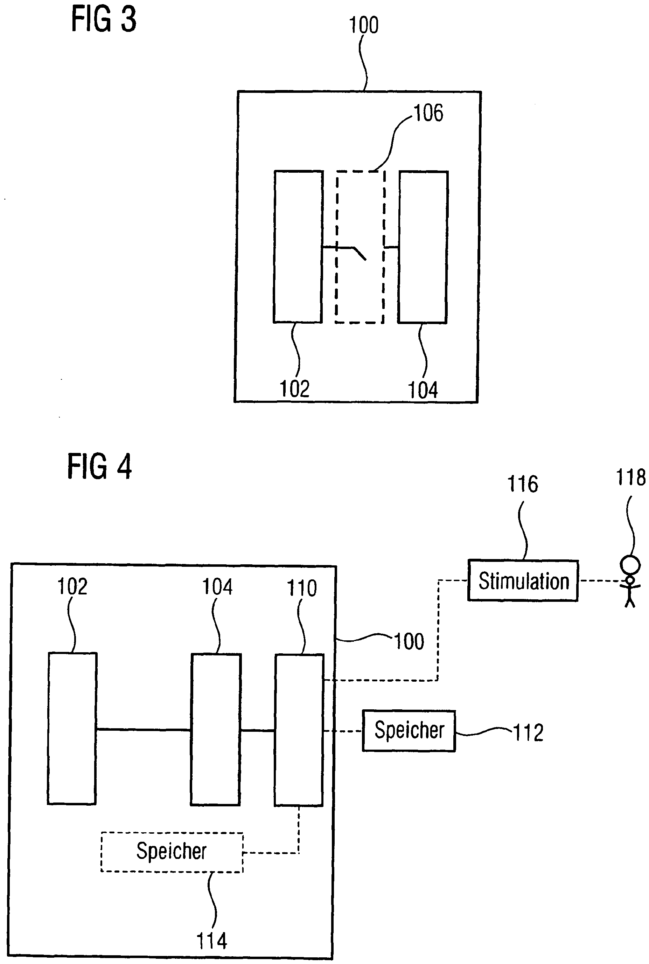

Fig. 3 zeigt ein Beispiel einer Vorrichtung zum Nachweis von Apnoen 100, welches eine Bereitstellungseinrichtung 102 zum Bereitstellen eines photoplethysmografisch erfassten Pulswellensignals und eine Auswerteeinrichtung 104 zum Auswerten des Signals umfasst. Die Auswerteinrichtung 104 schließt auf das Vorhandensein oder den Beginn einer Apnoe, wenn eine Änderung eines niederfrequenten Anteils des Pulswellensignals, welches von der Bereitstellungseinrichtung 102 bereit gestellt wurde, ein vorbestimmtes Nachweiskriterium erfüllt. Beispiele für solche Nachweiskriterien sind anhand der vorgehenden Figuren diskutiert worden.

Die Bereitstellungseinrichtung 102 kann beispielsweise das in Echtzeit von einem Photoplethysmografiesensor aufgenommene Pulswellensignal empfangen, um dieses bereitzustellen. Alternativ ist möglich, dass die Bereitstellungseinrichtung bereits aufgezeichnete Signale von einem externen Medium bezieht beziehungsweise von einem Speicher einliest, um diese bereitzustellen. In Fig. 3 ist darüber hinaus ein optionaler Tiefpassfilter 106 gezeigt, um den niederfrequenten Anteil des Pulswellensignals aus dem Pulswellensignal, welches von der Bereitstellungseinrichtung 106 bereitgestellt wird, zu erzeugen.

Dabei ist bevorzugt die Trennfrequenz des Tiefpassfilters derart gewählt, dass sie unterhalb der Pulsfrequenz eines Menschen liegt. Die Trennfrequenz kann daher somit unterhalb von einem Hz, unterhalb von 0,5 Hz oder auch unterhalb von 0,2 Hz gewählt werden.

Bei einer weiteren Ausführungsform der Erfindung wird die Trennfrequenz adaptiv angepasst, da die Auswerteinrichtung 104 ferner ausgebildet ist, das Pulswellensignal auf die Pulsfrequenz hin auszuwerten, um in Kenntnis der aktuellen Pulsfrequenz den Tiefpassfilter adaptiv derart zu steuern, dass die Tiefpassfilterung nicht durch die Pulsfrequenz beeinträchtigt wird, weil sichergestellt werden kann, dass die Grenzfrequenz des Tiefpassfilters immer unterhalb der Pulsfrequenz befindlich ist.

Der Tiefpassfilter 106 kann in weiteren Ausführungsformen ebenfalls durch eine Mittelwertbildung innerhalb eines Zeitintervalls ersetzt werden oder jedwede andere Maßnahme, die in der Lage ist, den niederfrequenten Signalanteil des Pulswellensignals, welches von der Bereitstellungseinrichtung 102 bereitgestellt wird, zu extrahieren.

Fig. 4 zeigt ein weiteres Ausführungsbeispiel der vorliegenden Erfindung in einer Vorrichtung zum Nachweis von Apnoen 100, welche ebenso wie die in Fig. 3 gezeigte Vorrichtung eine Bereitstellungseinrichtung 102 und eine Auswerteinrichtung 104 aufweist, so dass im Folgenden auf eine erneute Beschreibung der beiden Einrichtungen verzichtet wird. Insbesondere sind im gesamten Kontext dieser Anmeldung auch sämtliche funktionsidentischen oder funktionsähnlichen Komponenten mit denselben Bezugszeichen versehen, wobei deren Beschreibung anhand der einzelnen Ausführungsbeispiele wechselseitig aufeinander anwendbar ist beziehungsweise zwischen einzelnen Ausführungsbeispielen übertragen werden kann.

Die Vorrichtung zum Nachweis von Apnoen 100 weist ferner eine Alarmeinrichtung 110 auf, die ausgebildet ist, eine Alarmaktion auszuführen, wenn die Auswerteinrichtung auf das Vorhandensein einer Apnoe beziehungsweise auf den Beginn einer Apnoe geschlossen hat.

Dabei sind in Fig. 4 mehrere Alternativen für eine Alarmaktion dargestellt, welche selbstverständlich auch gleichzeitig ergriffen werden können. Ein Beispiel einer Alarmaktion ist dabei beispielsweise das Speichern des Zeitpunkts der nachgewiesenen Apnoe in einem externen Speicher 112 oder einem internen Speicher 114, welcher sich innerhalb der Vorrichtung zum Nachweis von Apnoen befinden kann.

Gleichzeitig ist es auf vorteilhafte Art und Weise möglich, ein Maß der Intensität der Apnoe zu speichern, welches basierend auf dem Nachweiskriterium ermittelt werden kann. Kommt als Nachweiskriterium beispielsweise ein Schwellwertvergleich zum Einsatz, so kann die Größe des Abweichens von dem Schwellwert als Maß für die Schwere der nachgewiesenen Apnoe verwendet werden.

Da, wie bereits erwähnt, basierend auf den photoplethysmografischen Signalen auch der Beginn einer Apnoe detektiert werden kann, wird bei weiteren Ausführungsbeispielen der vorliegenden Erfindung als Alarmaktion ein Stimulationssignal erzeugt, welches eines externe Stimulationseinrichtung 116 veranlassen kann, einen CPAP Gerät geeignet anzusteuern, sodass der Patient keine zu hohe Sauerstoffschuld eingeht.

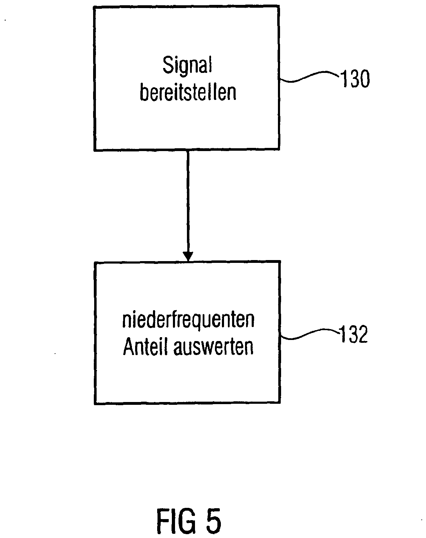

Fig. 5 zeigt ein Beispiel für ein Verfahren zum Nachweis von Apnoen, bei dem in einem Bereitstellungsschritt 130 photoplethysmografisch erfasste Pulswellensignale bereitgestellt werden. Diese werden in einem Analyseschritt beziehungsweise in einem Auswerteschritt 132 ausgewertet, um auf das Vorhandensein einer Apnoe zu schließen, wenn eine Änderung eines niederfrequenten Anteils des Pulswellensignals ein vorbestimmtes Nachweiskriterium erfüllt.

Zusammengefasst ist es also mit der vorliegenden Erfindung möglich, aus einer photoplethysmografisch erfassten Pulswelle beziehungsweise einem photoplethysmografisch erfassten Signal die schlafbezogenen Atmungsstörungen zu detektieren (die sogenannten Schlafapnoen).

Dabei ist es im praktischen Betrieb besonders vorteilhaft, dass bei einer polysomnografischen Schlafuntersuchung die Sauerstoffsättigung normalerweise routinemäßig mittels eines photoplethysmografischen Sensors erfasst wird. Auch viele andere Sensorsätze, die nur eine Teilmenge des vollständigen Polysomnografiedatensatzes aufzeichnen, schließen die SpO₂-Messung ein. Im klinischen Alltag können somit ohne die Notwendigkeit, einen zusätzlichen Sensor zu befestigen beziehungsweise zu kaufen, Apnoen nunmehr zuverlässig nachgewiesen werden. Darüber hinaus kann durch Anwendung des erfindungsgemäßen Konzepts eine Vielzahl anderer Sensoren, die bis dato bei der Polysomnografie verwendet werden, überflüssig werden, beziehungsweise diese Sensoren können zur Redundanzbildung verwendet werden.

Abhängig von den Gegebenheiten kann das erfindungsgemäße Verfahren in Hardware oder in Software implementiert werden. Die Implementierung kann auf einem digitalen Speichermedium, insbesondere einer Diskette oder CD mit elektronisch auslesbaren Steuersignalen erfolgen, die so mit einem programmierbaren Computersystem zusammenwirken können, dass das erfindungsgemäße Verfahren ausgeführt wird. Allgemein besteht die Erfindung somit auch in einem .Computer-Programm-Produkt mit einem auf einem maschinenlesbaren Träger gespeicherten Programmcode zur Durchführung des erfindungsgemäßen Verfahrens, wenn das Computer-Programm-Produkt auf einem Rechner abläuft. In anderen Worten ausgedrückt kann die Erfindung somit als ein Computer-Programm mit einem Programmcode zur Durchführung des Verfahrens realisiert werden, wenn das Computer-Programm auf einem Computer abläuft.

## Patentansprüche

1. Vorrichtung zum Nachweis von Apnoen mit folgenden Merkmalen:
einer Bereitstellungseinrichtung (102) zum Bereitstellen eines blutdruckabhängigen Pulswellensignals (20); **gekennzeichnet durch** :
eine Auswerteeinrichtung (104) zum Auswerten des bereitgestellten Pulswellensignals (20), um auf das Vorhandensein einer Apnoe zu schließen, wenn ein niederfrequenter Anteil (22; 30) des Pulswellensignals (20) unter einen zeitlich variablen Schwellwert (38) abfällt,
wobei die Auswerteeinrichtung (104) ausgebildet ist, um den zeitlich variablen Schwellwert (38) für einen Auswertezeitpunkt (32) **durch** Mittelung des niederfrequenten Anteils (22; 30) des Pulswellensignals (20) innerhalb eines zeitlich vor dem Auswertezeitpunkt (32) liegenden Zeitintervalls (36) zu bestimmen.

2. Vorrichtung gemäß Anspruch 1, bei der die Bereitstellungseinrichtung (102) ausgebildet ist, ein photoplethysmografisch erfasstes Pulswellensignal (20) bereit zu stellen.

3. Vorrichtung gemäß Anspruch 1 oder 2, mit folgendem zusätzlichen Merkmal:
einem Tiefpassfilter, um den niederfrequenten Anteil (22; 30) des Pulswellensignals (20) aus dem Pulswellensignal (20) abzuleiten.

4. Vorrichtung gemäß Anspruch 3, bei der der Tiefpassfilter eine Grenzfrequenz aufweist, die unterhalb einer Pulsfrequenz liegt.

5. Vorrichtung gemäß Anspruch 3, bei der Auswerteeinrichtung (104) ausgebildet ist, das Pulswellensignal (20) auf die Pulsfrequenz hin auszuwerten, um die Grenzfrequenz des Tiefpassfilters derart zu steuern, dass diese unterhalb der Pulsfrequenz liegt.

6. Vorrichtung gemäß einem Ansprüche 1 bis 5, bei der die Bereitstellungseinrichtung (102) einen Photoplethysmografiesensor umfasst.

7. Vorrichtung gemäß Anspruch 6, bei der der Photoplethysmografiesensor ausgebildet ist, Licht eines einzigen Wellenlängenbereichs zu verwenden.

8. Vorrichtung gemäß einer der Ansprüche 1 bis 7, bei der die Bereitstellungseinrichtung (102) ein Speicherinterface zum Auslesen eines auf einem externen Medium gespeicherten Pulswellensignals (20) aufweist.

9. Vorrichtung gemäß einem der Ansprüche 1 bis 8, bei der die Auswerteeinrichtung (104) einen Differenzierer umfasst, um eine zeitliche Ableitung des niederfrequenten Anteils (22; 30) des Pulswellensignals (20) zu bestimmen, wobei die Auswerteeinrichtung (104) ausgebildet ist, um auf das Vorhandensein einer Apnoe zu schließen, wenn die zeitliche Ableitung unter den vorbestimmten Schwellwert (38) abfällt.

10. Vorrichtung gemäß einem der Ansprüche 1 bis 9, mit einer Alarmeinrichtung (110), um eine Alarmaktion auszuführen, wenn die Auswerteeinrichtung (104) auf das Vorhandensein einer Apnoe geschlossen hat.

11. Vorrichtung gemäß Anspruch 10, bei der die Alarmeinrichtung (110) ausgebildet ist, als Alarmaktion das Speichern des Zeitpunkts des Nachweises einer Apnoe in einem externen Speicher durchzuführen.

12. Vorrichtung gemäß Anspruch 10 oder 11, bei der die Alarmeinrichtung (110) ausgebildet ist, als Alarmaktion ein Maß für die Schwere der nachgewiesenen Apnoe basierend auf dem Nachweiskriterium zu speichern.

13. Vorrichtung gemäß einem der Ansprüche 10 bis 12, bei der die Alarmeinrichtung (110) ausgebildet ist, als Alarmaktion eine Beatmungsunterstützungseinrichtung (116) zu aktivieren oder deren Unterstützungsfunktion zu erhöhen.

14. Verfahren zum Nachweis von Apnoen mit folgenden Schritten:
Bereitstellen eines blutdruckabhängigen Pulswellensignals (20) mittels einer Bereitstellungseinrichtung; und
Auswerten des bereitgestellten Pulswellensignals (20), um auf das Vorhandensein einer Apnoe zu schließen, wenn ein niederfrequenter Anteil (22; 30) des Pulswellensignals (20) unter einen zeitlich variablen Schwellwert (38) abfällt,
wobei der zeitlich variable Schwellwert (38) für einen Auswertezeitpunkt (32) durch Mittelung des niederfrequenten Anteils (22; 30) des Pulswellensignals (20) innerhalb eines zeitlich vor dem Auswertezeitpunkt (32) liegenden Zeitintervalls (36) bestimmt wird.

15. Computerprogramm mit einem Programmcode zur Durchführung des Verfahrens nach Anspruch 14, wenn das Programm auf einem Computer abläuft.

## Claims

1. An apparatus for detecting apneas, comprising:
a provision means (102) for providing a blood pressure-dependent pulse wave signal (20);
**characterized by**:
an evaluation means (104) for evaluating the pulse wave signal (20) provided so as to infer the existence of an apnea when a low-frequency portion (22; 30) of the pulse wave signal (20) falls below a temporally variable threshold value (38),
the evaluation means (104) being configured to determine the threshold value (38) for an evaluation time (32) by averaging the low-frequency portion (22; 30) of the pulse wave signal (20) within a time interval (36) which comes before the evaluation time (32).

2. The apparatus as claimed in claim 1, wherein the provision means (102) is configured to provide a photoplethysmographically detected pulse wave signal (20).

3. The apparatus as claimed in claim 1 or 2, further comprising:
a low-pass filter for deriving the low-frequency portion (22; 30) of the pulse wave signal (20) from the pulse wave signal (20).

4. The apparatus as claimed in claim 3, wherein the low-pass filter comprises a cutoff frequency which is below a pulse frequency.

5. The apparatus as claimed in claim 3, wherein the evaluation means (104) is configured to evaluate the pulse wave signal (20) in terms of the pulse frequency so as to control the cutoff frequency of the low-pass filter such that said cutoff frequency is below the pulse frequency.

6. The apparatus as claimed in any of claims 1 to 5, wherein the provision means (102) comprises a photoplethysmography sensor.

7. The apparatus as claimed in claim 6, wherein the photoplethysmography sensor is configured to employ light of a single wavelength range.

8. The apparatus as claimed in any of claims 1 to 7, wherein the provision means (102) comprises a memory interface for reading out a pulse wave signal (20) stored on an external medium.

9. The apparatus as claimed in any of claims 1 to 8, wherein the evaluation means (104) comprises a differentiator so as to determine a temporal derivation of the low-frequency portion (22; 30) of the pulse wave signal (20), the evaluation means (104) being configured to infer the existence of an apnea when the temporal derivation falls below the predetermined threshold value (38).

10. The apparatus as claimed in any of claims 1 to 9, comprising an alarm means (110) so as to perform an alarm action when the evaluation means (104) has inferred the existence of an apnea.

11. The apparatus as claimed in claim 10, wherein the alarm means (110) is configured to store, as an alarm action, the time of the detection of an apnea in an external memory.

12. The apparatus as claimed in claim 10 or 11, wherein the alarm means (110) is configured to store, as an alarm action, a measure of the severity of the detected apnea on the basis of the detection criterion.

13. The apparatus as claimed in any of claims 10 to 12, wherein the alarm means (110) is configured to activate, as an alarm action, a ventilation support means (116), or to increase the support function thereof.

14. A method of detecting apneas, comprising:
providing a blood pressure-dependent pulse wave signal (20) by means of a provision means; and
evaluating the pulse wave signal (20) provided so as to infer the existence of an apnea when a low-frequency portion (22; 30) of the pulse wave signal (20) falls below a temporally variable threshold value (38),
the threshold value (38) for an evaluation time (32) being determined by averaging the low-frequency portion (22; 30) of the pulse wave signal (20) within a time interval (36) which comes before the evaluation time (32).

15. A computer program comprising a program code for performing the method as claimed in claim 14, when the program runs on a computer.

## Revendications

1. Dispositif pour détecter des apnées, aux caractéristiques suivantes:
un moyen de mise à disposition (102) destiné à mettre à disposition un signal d'onde de pouls fonction de la tension artérielle (20);
**caractérisé par**:
un dispositif d'évaluation (104) destiné à évaluer le signal d'onde de pouls mis à disposition (20), pour conclure de la présence d'une apnée lorsqu'une part de basse fréquence (22; 30) du signal d'onde de pouls (20) tombe au-dessous d'une valeur de seuil variable dans le temps (38),
dans lequel le moyen d'évaluation (104) est réalisé pour déterminer la valeur de seuil variable dans le temps (38) pour un moment d'évaluation (32) par réduction à la moyenne de la part de basse fréquence (22; 30) du signal d'onde de pouls (20) dans un intervalle de temps (36) situé dans le temps avant le moment d'évaluation (32).

2. Dispositif selon la revendication 1, dans lequel le moyen de mise à disposition (102) est réalisé pour mettre à disposition un signal d'onde de pouls (20) détecté de manière photopléthysmografique.

3. Dispositif selon la revendication 1 ou 2, à la caractéristique additionnelle suivante:
un filtre passe-bas, pour dériver du signal d'onde de pouls (20) la part de basse fréquence (22; 30) du signal d'onde de pouls (20).

4. Dispositif selon la revendication 3, dans lequel le filtre passe-bas présente une fréquence limite qui se situe au-dessous de la fréquence de pouls.

5. Dispositif selon la revendication 3, dans lequel le moyen d'évaluation (104) est réalisé pour évaluer le signal d'onde de pouls (20) quant à la fréquence de pouls, pour régler la fréquence limite du filtre passe-bas de sorte que cette dernière se situe au-dessous de la fréquence de pouls.

6. Dispositif selon l'une des revendications 1 à 5, dans lequel le moyen de mise à disposition (102) comporte un capteur photopléthysmografique.

7. Dispositif selon la revendication 6, dans lequel le capteur photopléthysmografique est réalisé pour utiliser la lumière d'une seule plage de longueurs d'onde.

8. Dispositif selon l'une des revendications 1 à 7, dans lequel le moyen de mise à disposition (102) présente une interface de mémoire destinée à lire un signal d'onde de pouls (20) mémorisé sur un support externe.

9. Dispositif selon l'une des revendications 1 à 8, dans lequel le moyen d'évaluation (104) comporte un différenciateur, pour déterminer une dérivation temporelle de la part de basse fréquence (22; 30) du signal d'onde de pouls (20), le moyen d'évaluation (104) étant réalisé pour conclure de la présence d'une apnée lorsque la dérivation temporelle tombe au-dessous de la valeur de seuil prédéterminée (38).

10. Dispositif selon l'une des revendications 1 à 9, avec un moyen d'alarme (110), pour réaliser une action d'alarme lorsque le moyen d'évaluation (104) a conclu de la présence d'une apnée.

11. Dispositif selon la revendication 10, dans lequel le moyen d'alarme (110) est réalisé pour effectuer, comme action d'alarme, la mémorisation du moment de détection d'une apnée dans une mémoire externe.

12. Dispositif selon la revendication 10 ou 11, dans lequel le moyen d'alarme (110) est réalisé pour mémoriser, comme action d'alarme, une mesure de la gravité de l'apnée détectée sur base du critère de détection.

13. Dispositif selon l'une des revendications 10 à 12, dans lequel le moyen d'alarme (110) est réalisé pour activer, comme action d'alarme, un moyen d'assistance de détermination (116) ou augmenter sa fonction d'assistance.

14. Procédé pour détecter des apnées, aux étapes suivantes consistant à:
mettre à disposition un signal d'onde de pouls fonction de la tension artérielle (20) au moyen d'un moyen de mise à disposition;
et
évaluer le signal d'onde de pouls mis à disposition (20), pour conclure de la présence d'une apnée lorsqu'une part de basse fréquence (22; 30) du signal d'onde de pouls (20) tombe au-dessous d'une valeur de seuil (38) variable dans le temps,
dans lequel la valeur de seuil (38) variable dans le temps pour un moment d'évaluation (32) est déterminée par réduction à la moyenne de la part de basse fréquence (22; 30) du signal d'onde de pouls (20) dans un intervalle de temps (36) situé dans le temps avant le moment d'évaluation (32).

15. Programme d'ordinateur avec un code de programme pour réaliser le procédé selon la revendication 14 lorsque le programme est exécuté sur un ordinateur.
